(19) Europäisches Patentamt
European Patent Office
Office européen des brevets

(11) Publication number: **0 406 489 A1**

(12) **EUROPEAN PATENT APPLICATION**

(21) Application number: 89306740.5

(22) Date of filing: 03.07.89

(51) Int. Cl.⁵: **A61B 1/32**

(43) Date of publication of application:
09.01.91 Bulletin 91/02

(84) Designated Contracting States:
DE FR GB

(71) Applicant: Lee, Chenault D.
57 Grattan Court
Wanniassa Australian Capital Territory
2903(AU)

(72) Inventor: Lee, Chenault D.
57 Grattan Court
Wanniassa Australian Capital Territory
2903(AU)

(74) Representative: Hartley, David et al
Withers & Rogers 4 Dyer's Buildings
Holbornorn
London, EC1N 2JT(GB)

(54) **Disposable vaginal speculum.**

(57) A disposable speculum of synthetic plastic material for vaginal inspection is described. The speculum (1) comprises two dilation blade members (2, 3) which are attached to each other and may be moved apart. One of the blades (3) has an extended handle portion (3a) which is so constructed that the included angle (5) between the handle and the dilation blade is not less than 100 degrees.

EP 0 406 489 A1

## FIELD OF THE INVENTION

This invention relates to the class of device known as a speculum, in particular that type of speculum used for inspection of vaginal walls and the cervix uteri.

## DESCRIPTION OF THE PRIOR ART

Generally, prior art speculum construction has been of metal: the structure comprised two elongate dilator blade portions which could be moved apart or together on a pivotal or lost motion type of hinge, the movement being performed by a pair of hand-grip sections which were extensions of the blade portions. One problem with the use of metal construction is the difficulty of sterilization for re-use. This can only be properly done in an autoclave, and few general practitioners have such equipment available in their surgeries. Another problem is that metal feels cold and uncomfortable to the patient, so a metal speculum has to be warmed before use. In order to overcome these problems, it has been proposed to construct a cheap disposable speculum from a synthetic plastics material. These devices incorporate a slot and ratchet device as distinct from the metal constructed devices which use a thumb screw or the like, for the purpose of locking the blades of the speculum into a particular relative position. However, the plastic disposable devices still suffer from the defect of the difficulty of insertion, unless the patient is supported on special equipment such as a surgical table, again an item not necessarily available in the average general practitioner's surgery.

## SUMMARY OF THE INVENTION

The applicant in respect of the present invention has discovered that the aforesaid problem can be overcome if the angle between the dilator blade portions and the handle portions is made no less than 100 degrees.

Broadly, the invention comprises a vaginal speculum comprising a pair of elongate dilator blade members pivotally attached to each other whereby to be movable from a position in which they are mutually adjacent to positions in which they are spread apart, one of said blade members having a rearward extension forming an elongate handle portion, the included angle between the longitudinal axis of said handle portion and the longitudinal axis of said pair of dilator blades in closed position being not less than 100 degrees, said speculum being constructed of synthetic plastics material.

## BRIEF DESCRIPTION OF THE DRAWINGS

A better understanding of the invention will be gained by referring to the accompanying drawings in which:
Figure 1 is a side elevation of the speculum of the invention;

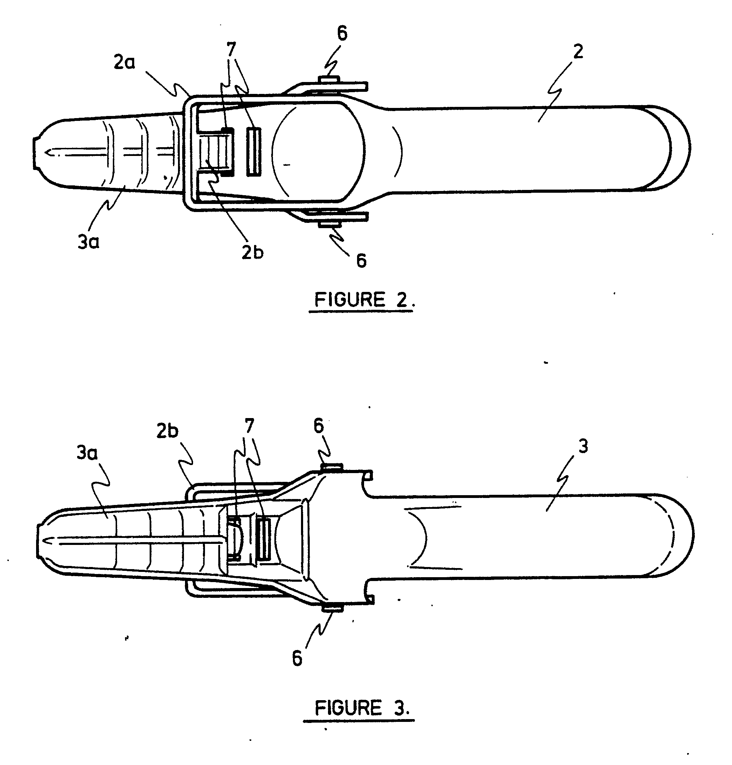

Figure 2 is a plan view thereof;
Figure 3 is an underside (plan) view; and
Figures 4 and 5 are front and rear elevations respectively.

## DETAILED DESCRIPTION

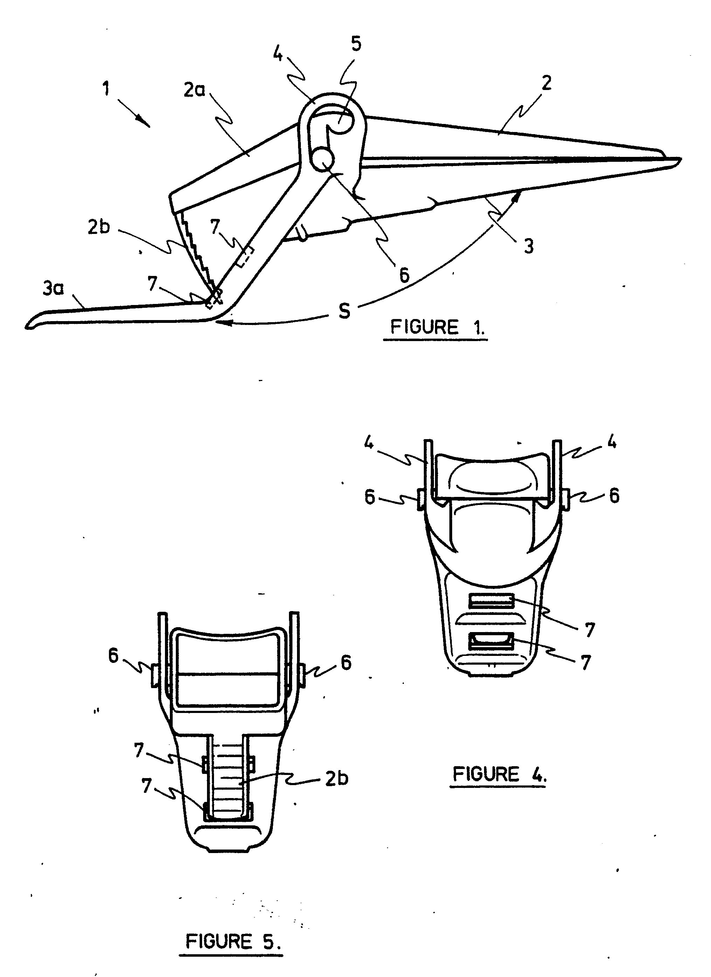

Referring to Figure 1, a speculum 1 comprises dilator blade portions 2 and 3. Upper blade portion 2 has a rear extension 2a to which is attached ratchet member 2b. Blade portion 3 has a rear extension 3a, including a pair of slots therein 7 for receiving member 2b whereby to lock the positions of blade portions 2 and 3 relative to each other. Blade portion 3 includes a pair of projections 4 each of which has a double slot 5 therein, for respectively receiving pivot pins 6 forming part of blade portion 2. Thus if pins 6 are in the rear (longer) of the two slots, ratchet member 2b will engage in the rearmost of the two slots 7, and if the pins are in the forward slot, ratchet member 2 engages in the foremost of the two slots 7.

In operation, blade portions 2, 3 (in closed position as shown) are inserted into the patient. The practitioner grips rear extension 3a which constitutes a handle member. Once the device is inserted, the practitioner presses members 2a and 3a together, to spread the blade portions 2 and 3 and to engage the ratchet member 2 into one of the slots 7, as appropriate. This distends the walls of the vagina so that they are more readily visible, and also makes visible the cervical area; it will be noted that the device is locked in this open position by the ratchet arrangement. It is important to note that the angle S degrees between extension 3a and the median axis of the dilator blade portions is no less than 100 degrees. As shown, the angle is almost 180 degrees i.e. the two axes are almost parallel. Unless this is so, the problem of the prior art referred to above is not avoided. It is preferred also - for a standard construction speculum - that the spread between the tips of members 2 and 3 does not exceed 4 centimetres before the first notch of the ratchet mechanism is engaged.

As a throw-away device, it is essential that the speculum be constructed of synthetic plastics material, preferably a transparent material such as polymethyl methacrylate (perspex or lucite) or polystyrene. (Being transparent enables the better transmission and distribution of light for internal viewing; while there is usually a separate lighting arrangement, a detachable lighting device can be affixed directly to the speculum.)

If desired, the dilator blades may be precoated with a suitable lubricant.

Thus, what has been described is a convenient to use disposable, light, cheap speculum, which has none of the problems of the prior art devices and proposals.

## Claims

1. A vaginal speculum comprising a pair of elongate dilator blade members pivotally attached to each other whereby to be movable from a position in which they are mutually adjacent to positions in which they are spread apart, one of said blade members having a rearward extension forming an elongate handle portion, the included angle between the longitudinal axis of said handle portion and the longitudinal axis of said pair of dilator blades in closed position being not less than 100 degrees, said speculum being constructed of synthetic plastics material.

2. A speculum as defined in claim 1, wherein one of said dilator blade members includes a pair of spaced projections, each having an aperture therein, the other of said blade members sitting between said projections and including pin means engaged in said apertures, whereby to constitute said pivotal attachment.

3. A speculum as defined in claims 1 or 2 including locking means to retain said dilator blade members in selected spread apart positions.

4. A speculum as defined in Claim 3, wherein said elongate handle portion has an aperture in a forward portion thereof, the other dilator blade having a rearward extending member including a plurally notched portion adapted to engage in said handle portion aperture, whereby to constitute said locking means.

5. A speculum as defined in any preceding claim, constructed of polymethyl methacrylate.

FIGURE 1.

FIGURE 4.

FIGURE 5.

FIGURE 2.

FIGURE 3.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | US-A-3332414 (GASPER)<br>* figures 1-5 *<br>* column 3, line 30 - column 4, line 23 *<br>* column 5, line 36 - column 6, line 2 *<br>* column 6, line 64 - column 7, line 44 *<br>--- | 1-4 | A61B1/32 |
| X | FR-A-2610507 (CORNIER)<br>* figure 2 *<br>* page 2, line 33 - page 6, line 13 * | 1 | |
| A | | 3, 4 | |
| A | ---<br>US-A-3716047 (MOORE ET AL)<br>* figures 1-4, 8, 9 *<br>* column 2, line 29 - column 3, line 74 *<br>----- | 1-5 | |

**TECHNICAL FIELDS SEARCHED (Int. Cl.5 )**

A61B

The present search report has been drawn up for all claims

| Place of search | Date of completion of the search | Examiner |
|---|---|---|
| THE HAGUE | 06 MARCH 1990 | CHEN A.H. |